(19) **Europäisches Patentamt**
**European Patent Office**
**Office européen des brevets**

(11) **EP 1 830 730 B1**

(12) **EUROPEAN PATENT SPECIFICATION**

(45) Date of publication and mention
of the grant of the patent:
**02.03.2011 Bulletin 2011/09**

(51) Int Cl.:
***A61B 18/04*** (2006.01)

(21) Application number: **05849203.4**

(22) Date of filing: **09.12.2005**

(86) International application number:
**PCT/US2005/044547**

(87) International publication number:
**WO 2006/063202 (15.06.2006 Gazette 2006/24)**

(54) **DEVICE FOR TREATMENT OF SKIN CONDITIONS**

VORRICHTUNG ZUR BEHANDLUNG VON HAUTERKRANKUNGEN

DISPOSITIF DE TRAITEMENT D'ETATS CUTANES

(84) Designated Contracting States:
**AT BE BG CH CY CZ DE DK EE ES FI FR GB GR HU IE IS IT LI LT LU LV MC NL PL PT RO SE SI SK TR**

(30) Priority: **10.12.2004 US 634904 P**
**16.02.2005 US 653740 P**
**30.08.2005 US 216595**

(43) Date of publication of application:
**12.09.2007 Bulletin 2007/37**

(73) Proprietor: **Koninklijke Philips Electronics N.V.**
**5621 BA Eindhoven (NL)**

(72) Inventors:
• **DA SILVA, Luiz, B.**
**Danville, CA 94526 (US)**

• **CHOI, George**
**Redwood City, CA 94062 (US)**

(74) Representative: **Damen, Daniel Martijn et al**
**Philips Intellectual**
**Property & Standards**
**High Tech Campus 44**
**5656 AE Eindhoven (NL)**

(56) References cited:
**WO-A-89/04137     WO-A-96/35469**
**US-A- 4 860 744     US-A- 6 066 153**
**US-A- 6 162 211     US-B1- 6 176 856**
**US-B2- 6 635 075**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND OF THE INVENTION

Acne affects more than 90% of all adolescents, nearly 50% of all adult women and 25% of all adults. One of the main causes of acne is improper drainage of a hair follicle caused by a plug of dead cells or dirt that trap oil and bacteria. The hair follicle opening is approximately 50 $\mu$m to about 100 $\mu$m in diameter. The opening of any other pore on the skin is substantially smaller. In particular, the opening of a sweat pore is less than about 30 $\mu$m in diameter. There are a variety of ways to treat acne. Benzoyl peroxide is one of the most commonly used ingredients in over-the-counter treatments, and it can be very effective in treating mild cases of non-inflammatory acne. It is safe for children as well as adults, and may be combined with other topical or oral treatments. For patients who suffer from moderate to severe acne, doctors may prescribe a combination of topical remedies and oral antibiotics. The most common oral medications used to treat acne are tetracycline, minocycline, doxycycline and erythromycin. Alternatives to medication include UV light radiation, laser treatment, or abrasion. Most of these systems are large and in most cases require professional treatment. U.S. Patent No. 6,635,075 by Li et al. describes a heating device that can also be used to treat acne. The device described therein uses a heater and temperature sensor to maintain a constant temperature surface that can be applied to skin. In order to prevent burns during the long application time (minutes), the maximum temperature allowed is about 62[deg.]C. The long treatment time makes this device impractical for normal acne treatment. A need exists for a compact device that can be used effectively and quickly to treat acne. The present invention fulfills this need, and further provides related advantages.

Methods of rejuvenating skin range from the aggressive face lift surgery and skin resurfacing by lasers (for example, using CO2 lasers) or chemical peel, to the less effective non-ablative lasers systems, RF energy skin rejuvenation systems, microdermabrasion, other abrasive devices as well as various lotions and creams.

Additional alternative methods include the application of UV, infrared and light radiation, laser treatment, mechanical abrasion or ultrasound energy. Most of these systems are large and in most cases require professional treatment. A need exists for effective skin rejuvenation devices.

SUMMARY OF THE INVENTION

It is an object of the present invention to provide devices for treating skin conditions and skin lesions, such as acne, removing fine wrinkles and clearing skin. Another object of the present invention is to provide a hand held device that can be safely used to heat tissue without causing a burn. These and other objects will be apparent to those skilled in the art based on the teachings herein.

One aspect of the invention provides a device for treatment of skin conditions including a heating element adapted and configured to contact a skin surface; and a controller adapted and configured to automatically heat the heating element to a temperature of at least about 70 deg. Celsius in a period of less than about one second and to maintain the heating element at a temperature of at least about 70 deg. Celsius for a period of less than one second in response to a user input and to keep the energy release to the skin below the threshold for tissue burn. In some embodiments, the controller is further adapted to cease heating the heating element and to reheat the heating element without a further user input. The heating element and controller may be located in a housing, with the housing being capable of being held in a hand of a user.

A further aspect of the invention is directed to a user interface, which is adapted and configured to be activated by a user to provide the user input. The controller may be further adapted to cease heating the heating element and to reheat the heating element without a further user input.

Yet a further aspect of the invention is directed to a heating circuit, which is adapted and configured to heat the heating element to a temperature of at least about 67 deg. Celsius for less than about 0.1 second.

In each of these aspects of the invention, the heating element could be a resistive heater, such as a thin metal resistor. The heating element may use a metal selected from the group consisting of nichrome, tungsten, aluminum, gold, copper and/or steel and may have a backing layer, an electrically insulating protective layer, and/or a high conductivity layer. A skin contact surface of the heating element may have a surface area of about 1 cm2.

In each of these aspects of the invention; the heating element maybe adapted to be heated to a of about 75 deg. Celsius - about 100 deg. Celsius or about 100 deg. Celsius - about 200 deg. Celsius in a period of less than about 0.5 seconds. The total energy transferred to the skin may be limited to less than about 50 J/cm2 or less than about 5 J/cm 2. The device may also include a temperature sensor adapted and configured to sense the temperature generated by the heating element and to control the temperature of said heating element. The device may also include a cooling element adapted and configured to cool the heating element and/or a skin surface to a temperature of less than about 60 deg Celsius. The device may also include a light emitting diode. In some embodiments, the heating circuit is further adapted to heat the heating element to a temperature of at least 67 deg Celsius for less than about 0.1 second, lower the temperature of the heating element for a period of about 0.5 seconds, and reheat the heating element to a temperature of at least 67[deg.]C for less than about 0.1 second. In some embodiments, activation of the user interface by a user heats the heating element to a peak temperature of about 250 deg. Celsius in about 1 millisecond and the peak temperature decays to about 100 deg. Celsius in less than about 0.5 seconds. It is described a method of treating a skin condition including the step of heating a skin surface of a subject to a temperature of at least about 70 deg. Celsius in less than about 1 second for a time duration of less than about 1 second. Some embodiments include the step of ceasing the heating step for about ten milliseconds to about five seconds, then reheating the skin surface to a temperature of at least about 70 deg. Celsius for a time duration of less than about 1 second. The ceasing and reheating steps maybe repeated.

It is also disclosed a method of operating a device for treating a skin condition by applying to a skin surface of a subject at least two pulses of heat to raise the skin surface to a peak temperature of at least about 70 deg. Celsius, the peak temperature being achieved by each pulse in a time period of less than about one second. Some embodiments include the step of providing a delay between the pulses of heat to allow the skin surface to reach a temperature of less applying a topical substance to the surface of said skin prior to, during, and/or after the application of peak temperature. The skin condition treated may be acne, a wart, a cold sore, and/or a skin -wrinkle. In some embodiments, the temperature stays above about 70 deg. Celsius for a period of less than about 1 second. Yet another aspect of the invention provides a method of operating a device for treating a skin condition comprising applying a heat source to a skin surface and heating the heat source to a peak temperature greater than about 67 deg. Celsius for less than about 0.1 second. Some embodiments include the further step of ceasing the heating step for about ten milliseconds to about five seconds, then reheating the skin surface to a temperature of at least about 70 deg. Celsius for a time duration of less than about 1 second. The ceasing and reheating steps may be repeated. In some embodiments, the heating element comprises a resistive heater and/or a thin metal resistor. The metal in the heating element can be nichrome, tungsten, aluminum, gold, copper and/or steel. The resistive heater is preferably less than 200 μm thick and has a surface area of about 1 cm2. The heating element includes in some embodiments a backing layer to add strength and to conduct heat; an electrically insulating protective layer for placement directly onto skin; a high conductivity layer for placement directly onto skin to improve temperature uniformity; and/or an electrical circuit configured to charge a capacitor that stores enough energy to heat said heating element. The controller may include a control circuit and a temperature sensor, wherein the control circuit monitors the temperature sensor and prevents the heating element from heating to a temperature that would burn human skin. In some embodiments, the device further comprises a cooling element. Typically the cooling element cools the skin and/or the heating element, preferably in between heat pulses. Further, the cooling element could ensure that the heating element is not again heated until it and/or the skin has reached a temperature of about 50 deg. Celsius or less. In other embodiments, the device further comprises a temperature sensor, such as a thermocouple, and or a battery powered light emitting diode attached to the housing to provide illumination.

Another aspect of the invention is a device for delivering a controlled amount of thermal energy to tissue comprising a resistive heating element, a circuit to deliver a fixed amount of energy to the resistive heating element, and an element to activate and trigger the circuit. Preferably, the device further comprises a thin insulating layer placed between the resistive heating element and the surface of the targeted skin. The device preferably heats the skin quickly to a temperature greater than 50 deg. Celsius. Another embodiment is a device for treating the skin comprising a thermoelectric cooler adapted and configured to contact a skin surface, a circuit to deliver a controlled amount of energy to said thermoelectric cooler, an element to activate and trigger the circuit. In a preferred embodiment, the device further comprises an element adapted and configured to reverse the polarity of the thermoelectric cooler so that after being heated for a period of time, the hot plate of the thermoelectric cooler becomes a cold plate, and is allowed to cool the surface of the skin for a second period of time. In one embodiment, the device further comprises a plurality of thermoelectric coolers so that a spatial and temporal heating and cooling configuration is tailored at the targeted skin surface. Preferably, the device heats the surface of the skin to a peak temperature in excess of about 50 deg. Celsius for a duration of about 0.5 second or less. In some embodiments, the devices described herein heat to a peak temperature of about 200 deg. Celsius and this peak temperature is maintained for less than about 5 seconds, preferably for less than about 1 seconds.

In one embodiment, the invention is a device for treatment of skin conditions comprising a heating element adapted and configured to heat to a peak temperature of at least about 70 deg. Celsius in a period of less than about one second, to maintain said peak temperature for a period of less than one second, and to contact a skin surface; and a controller adapted and configured to control the heat generated by said heating element in response to a user input. This device can further include a housing for the heating element and controller, said housing being capable of being held in the hand of a user.

In another embodiment, the invention is a device for treatment of skin conditions comprising a housing adapted and configured to be held in the hand of a user, said housing comprising a heating element adapted and configured to heat to a peak temperature of at least about 70 deg. Celsius in a period of less than about one second, to maintain said peak temperature for a period of less than one second, and to contact a skin surface; a controller adapted and configured to control the heat generated by said heating element in response to a user input; a power source; and a user interface adapted and configured to be managed by a user to control said heating element. Yet another embodiment of the invention is a device for treatment of skin conditions comprising a heating element adapted and configured to be placed against a skin surface to be treated; a handle area adapted and configured to be held by a user and to support the heating element when placed against the skin surface; and a heating circuit adapted and configured to heat the heating element to a peak temperature of at least about 67 deg. Celsius for less than about 0.1 second. The devices described herein can include a temperature sensor adapted and configured to sense a heat generated by said heating element and to control the heat generated by the heating element. They can also include a cooling element adapted and configured to cool said heating element and/or a skin surface to a temperature of less than about 60 deg. Celsius. In a preferred embodiment, the device includes a heating circuit adapted to heat the heating element to a temperature of at least 67 deg. Celsius for less than about 0.1 second, lower the temperature of the heating element for a period of about 0.5 seconds, and reheat the heating element to a temperature of at least 67 deg. Celsius for less than about 0.1 second. In another preferred embodiment, upon activation of the device by a user the heating element is heated to a peak temperature of about 250 deg. Celsius in about 1 millisecond and said peak temperature decays to about 100 deg. Celsius in less than about 0.5 seconds.

It is disclosed a method of operating a device for the treatment of skin conditions, such as acne, warts, and skin wrinkles. The device is used in combination with topical agents used in the treatment of skin conditions. This topical agent can be applied by the user or can be applied with the devices described herein. The present invention can also be combined with acne treatment creams and gels to further accelerate treatment. For example, creams or gels containing benzoyl peroxide could be applied before or after applying the device. It is disclosed a method of treating a skin condition comprising heating a skin surface of a subject to a peak temperature of at least about 70 deg. Celsius, wherein said peak temperature is achieved within a time period of less than about 1 second and said heating is for a time duration of less than about 1 second. It is disclosed a method of treating a skin condition comprising a peak temperature of about 250 deg. Celsius in about 1 millisecond and said peak temperature decays to about 100 deg. Celsius in less than about 0.5 seconds.

The device according to the invention can be used in combination with topical agents used in the treatment of skin conditions. This topical agent can be applied by the user or can be applied with the devices described herein. The device according to the invention can also be combined with acne treatment creams and gels to further accelerate treatment. For example, creams or gels containing benzoyl peroxide could be applied before or after applying the device. A preferred method of treating a skin condition comprising heating a skin surface of a subject to a peak temperature of at least about 70 deg. Celsius using the device according to the invention, wherein said peak temperature is achieved within a time period of less than about 1 second and said heating is for a time duration of less than about 1 second. Another preferred method is a method of treating a skin condition comprising applying to a skin surface of a subject at least two pulses of heat to raise the skin surface to a peak temperature of at least about 70 deg. Celsius, said peak temperature being achieved by each pulse in a time period of less than about one second. There can be a delay between the pulses of heat to allow the skin surface to reach a temperature of less than about 50 deg. Celsius. Preferably in the methods the peak temperature is maintained for a period of less than about 1 second. Another method of treating a skin condition comprising applying a heat source to a skin surface and heating the heat source to a temperature greater than about 67 deg. Celsius for less than about 0.1 second. This method can further comprise ceasing the heating step for a period of about 0.5 seconds and heating the heat source to a temperature greater than about 67 deg. Celsius for less than about 0.1 second.

Other objects and advantages of the present invention will become apparent from the following description and accompanying drawings.

The circuit will then recharge the capacitor and be ready to fire again within a few seconds. In order to reduce the risk of accidental burns, the heating element is allowed to cool before another heating pulse can be fired. When used in a multi-pulse mode, the device delivers multiple heat pulses automatically without further user input. In one embodiment, a temperature sensor (e.g., thermocouple) **34** monitors the temperature of the heating element and interacts with a controller on the circuit board 30 to prevent a second heating pulse until the temperature drops below an acceptable temperature (e.g., about 40°C). The thin resistive heater is typically made of metal (e.g., Nichrome (Nickel and Chromium alloy), tungsten, aluminum, copper, gold, steel) and is typically less than 200 $\mu$m thick. Suitable thin film resistors can also be found at Minco Products, Inc. (http://www.minco.com/) (e.g., Thermofoil™ heaters). Other suitable thin film resistors are available from Kyocera, Inc.

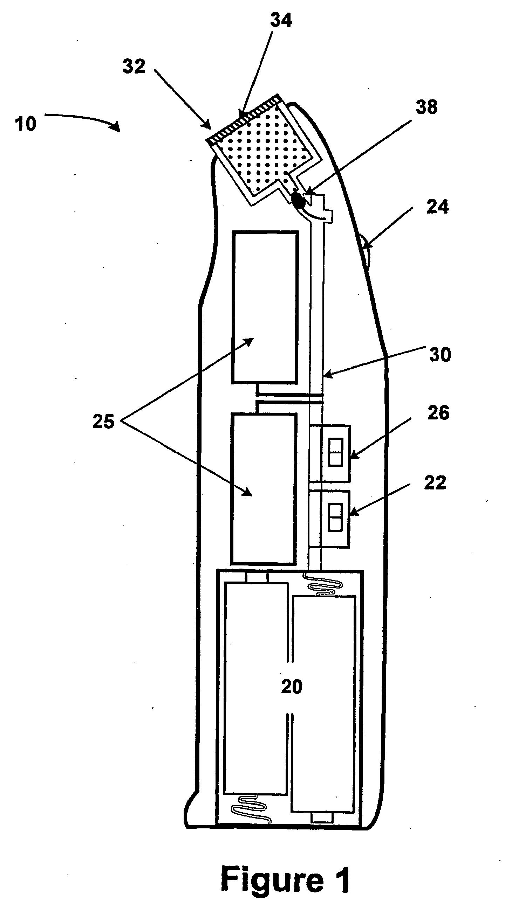

In one embodiment the user can select different power levels or multi-pulse heating formats. For example, as shown in Figure 1, a high and low power setting can be selected using button **26.** Alternatively, a single push button could be used to power the device and select operating modes. For example, pressing the button turns on the device at a low setting, pressing it again changes to high power setting, pressing it a second time changes to a multiple pulse heating format. Each time the button is pressed it cycles through all the possible modes. The current mode can be displayed by a series of LED's or a small LCD.

An optional LED **38** can also be integrated into the device to provide illumination and aid in treatment. For example blue and red light have been shown to treat acne. Rather than high/low, and off/on switches, the device can use a single button to select the desired operating mode. For example, pressing the button for more then 1 second turns device on or off. Pressing the button for a short time when on causes the device to switch the operating mode. This allows the device to operate in a wide variety of modes including complex multi-pulse sequences.

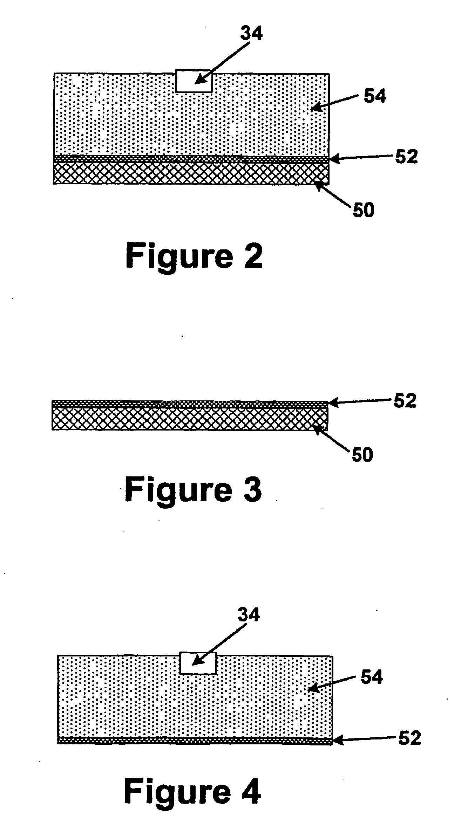

Figures 2, 3, and 4 show exemplary embodiments that may be substituted for the heating element **32**. A thick backing layer **54,** shown in figures 2 and 4, can be used to add strength to the heating element and also conduct heat away from the thin resistive heater **52.** In one embodiment, a thin protective layer **50** covers the resistive heater. In the preferred embodiment, the protective layer **50** is an electrical insulator and has good thermal conductivity. This protective layer **50** reduces the risk of shock to the user and can act to improve temperature uniformity across the surface of the heating element. Alternatively the thin resistive heater **52** can be chemically treated (e.g., anodized) to provide a very thin insulating layer to prevent electrical shock to the user. For most applications the thin resistive heater **52** and optional protective layer **50** are less than 500 $\mu$m thick to limit the total energy required to heat the material to the necessary peak temperature. This also limits the maximum energy that can be transferred into the tissue thereby reducing the risk of burns. A temperature sensor **34,** shown in figures 2 and 4, can be integrated into the backing layer **54** to monitor temperature. For most applications the surface area of the skin contacting surface of the heating element is approximately 1 cm$^2$.

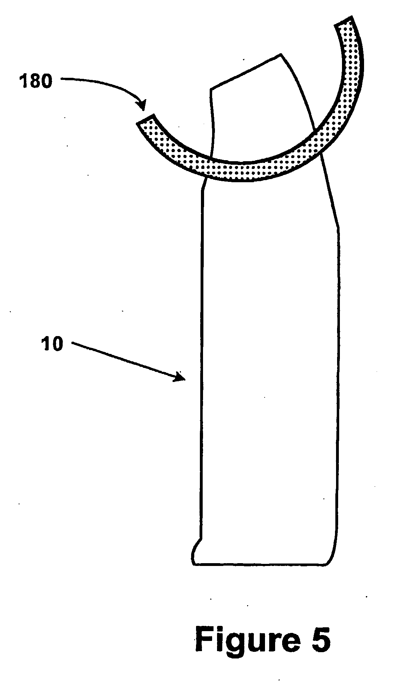

The heating element in the present invention will quickly cool by thermal conduction into tissue (and if present into the backing layer as well). The maximum energy that can be transferred to the skin is controlled by the peak temperature and the thickness of the heated layer. For example, for a 100 $\mu$m thick absorbing glass layer heated to 300°C the available energy to transfer to tissue that is at 30°C is approximately 5.7 J/ cm$^2$. For pulsed heating of a thin heater the cooling time can be estimated from the thermal relaxation time, $\tau = (\Delta x/\pi)^2 \rho\, c\, /\kappa$ where $\Delta x$ is the thickness, $\rho$ is the density, $c$ is the specific heat, and $\kappa$ is the thermal conductivity of heater. For glass, the relaxation time is approximately 1.2 msec. For a 100 $\mu$m thick copper layer heated to 300°C the available energy to transfer to tissue that is at 30°C is approximately 9.2 J/ cm$^2$. The relaxation time is approximately 8.65 $\mu$sec. By appropriate selection of the heating pulse format and heater materials the peak tissue temperature and time history can be controlled. Figure 5 shows another embodiment of the handheld acne treatment device that integrates a protective shield **180** to prevent the user from positioning the device on the eye.

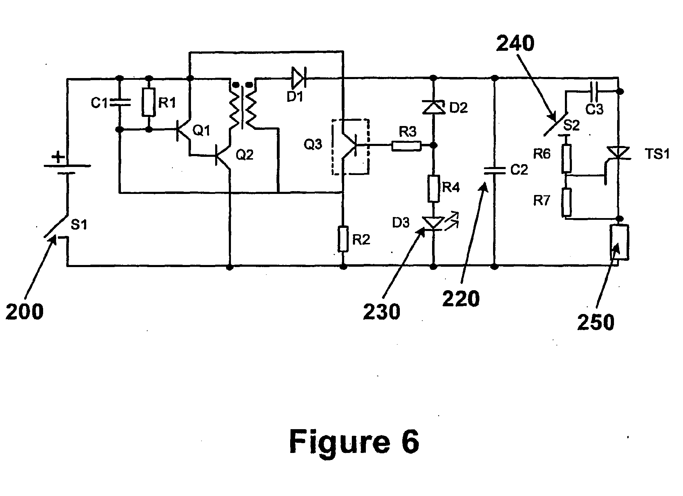

Figure 6 shows one possible circuit to pulse the thin resistive heater to the desired peak temperature. A switch **200** (S1) is turned on to activate the device and charge the capacitor **220** (C2). When the capacitor is fully charged, a lamp **230** LED (D3) turns on and the device is ready to fire. When the fire switch **240** (S2) is activated, it turns on the thyristor (TS1) and discharges the capacitor **220** through the thin resistive heater **250.** In a preferred embodiment the discharge through the thin resistive heater has a time constant of less than 10 ms. The capacitor **220** begins to charge again after firing and after several seconds (depending on battery and resistance) is fully charged. This circuit releases a maximum energy per pulse of ¼ CV$^2$ where C is the capacitor capacitance and V is the final voltage across the capacitor. By selecting appropriate values of C and V, the released energy can be kept below the threshold for tissue burns.

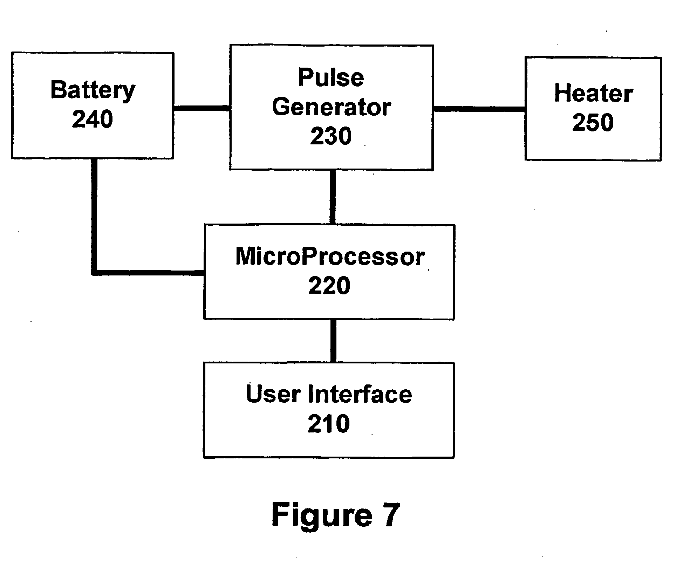

Figure 7 shows a block diagram of the key elements in the control electronics of an alternative circuit to control heating of the resistive heater. A user interface 710 that could include buttons, switches, or touch screens are used to select the operating mode of the device and generate a pulse. The user interface **710** is input into a microprocessor **720** (e.g. PIC16F676 by Microchip inc.) that controls all the functions of the pulse generator **730.** Power is provided by a battery **740** or directly from a power supply. The pulse generator **730** converts the voltage from the battery **740** to a high voltage that can be used to charge a capacitor that discharges through the heater **750** when the user presses a fire button. Typically the discharge through the thin resistive heater **750** has a time constant of less than 100 ms. A maximum energy per pulse of ½ CV$^2$ where C is the capacitor capacitance and V is the final voltage across the capacitor. By selecting appropriate values of C and V, the released energy can be kept below the threshold for tissue burns. The microprocessor **720** controls the charging and firing of the device directly based on user input. The user can select power and multi-pulse heating formats that can deliver heat deeper into tissue while preventing burns.

The possible pulse formats that can be produced include a series of constant energy pulses with a separation of less than about 2 seconds that could maintain a near constant temperature below the skin surface. A decaying pulse series with each successive pulse having a lower energy can also be used. An increasing pulse series with each successive pulse having a higher energy can also be used. By carefully adjusting the pulse series and per pulse energy, the peak tissue temperature and temperature profile inside tissue can be controlled to prevent burns. In preferred embodiments, the separation between pulses is greater than 10 ms but less than 5 seconds.

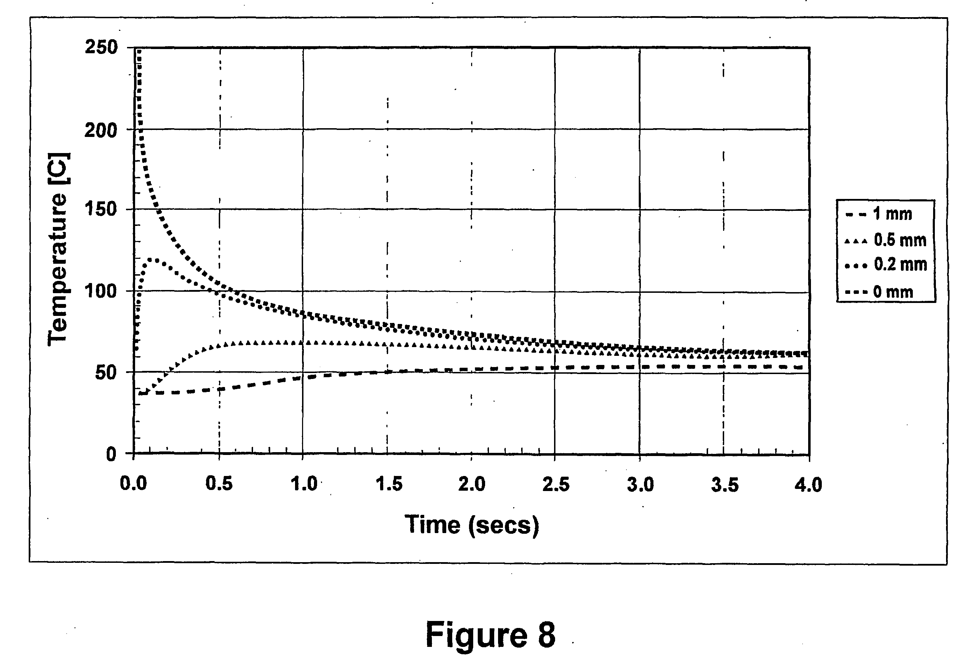

Figure 8 shows the calculated temperature history 0.2, 0.5 and 1.0 mm below the surface when a single heating pulse is applied at time equal 0. The high peak temperatures exist for less than 0.1 seconds which limits the risk of burns.

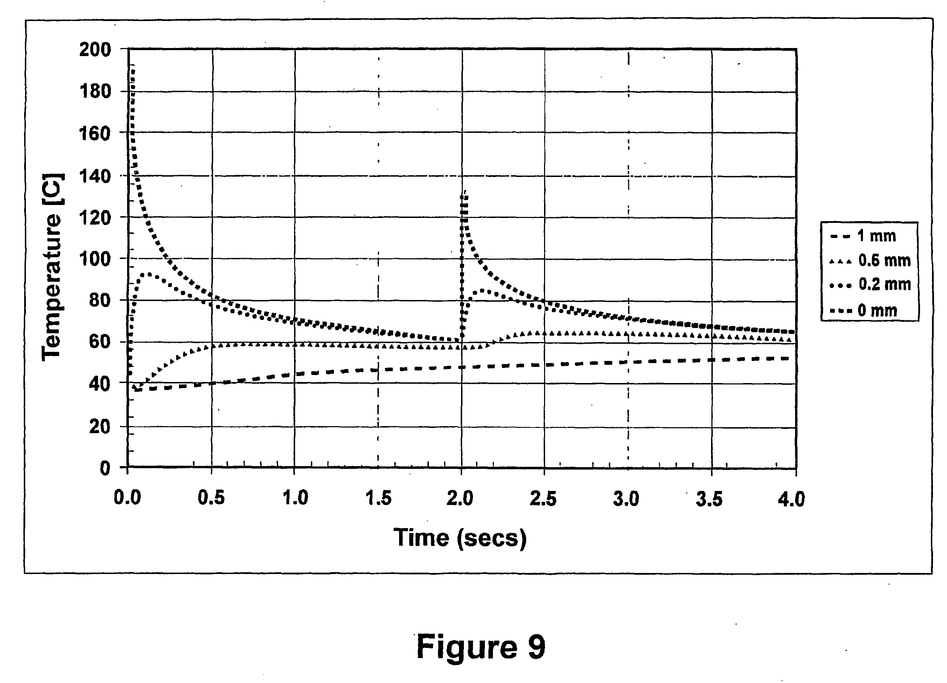

Figure 9 shows the calculated temperature history 0.2, 0.5 and 1.0 mm below the surface when two heating pulses are applied to the heater. The first pulse has higher total energy and produces a peak temperature of 180°C. Two seconds later a second pulse with one half of the energy of the first pulse reheats the thin metal heater. The resulting temperature inside the tissue is higher than that produced by the single pulse with an equal total energy. The multi-pulse capability allows the device to heat more tissue without exceeding the burn threshold which is a function of the peak temperature.

The short time duration of the high peak temperature is critical to preventing skin burns. Henriques (F.C. Henriques, "Studies of Thermal Injury: The Predictability and the Significance of Thermally Induced Rate Processes Leading to Irreversible Epidermal Injury", Archives of Pathology, 43, 5 May 1947, Pages 489-502) published a theory on skin burns based on a form of the Arrhenius equation for heat induced irreversible chemical reaction. Although numerous other studies have investigated the burn process, the conclusions are similar. A skin burn occurs as a result of thermally induced changes in protein structure that have an activation energy of about 600 MJ/kg-mol. For skin the Henriques Integral equation can be written as:

$$\omega = \int_0^t e^{226.78 - \frac{75000}{T}} dt$$

where T is the temperature in Kelvin at depth x and is a function of time, and $\omega$ is a function of burn injury. Integration is carried out over the time the basal layer temperature is greater then or equal to 44 °C. Second degree burns occur when $\omega = 1$. First degree burns occur for values of $\omega = 0.53$. Third degree burns occur at a critical value of $\omega = 1$ at the base of the dermis. For the present device under normal operation, $\omega < 0.4$ for depths greater than 100 $\mu$m below skin surface. For this reason the risk of burn is very low.

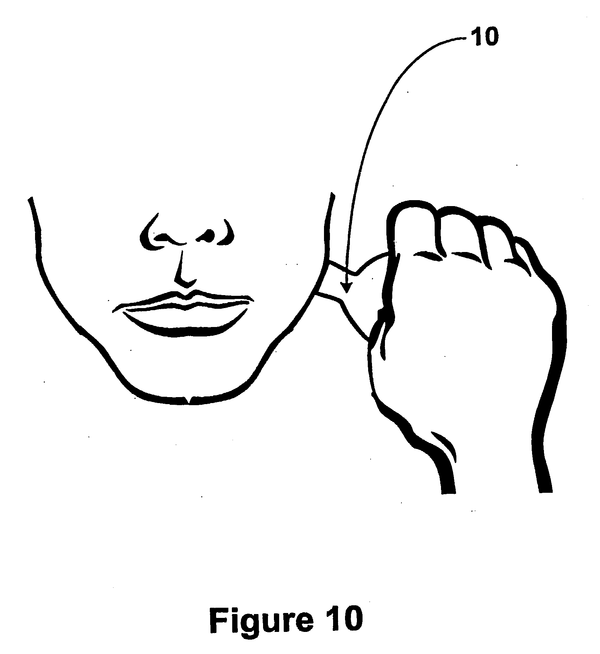

Figure 10 shows how the present invention would be used to treat a blemish on the face. The device **10** is activated and then placed in contact with the skin. When the device **10** is in good contact and fully charged, the fire button is pressed to deliver energy to the heating element, which then transfers its energy to the skin. The thermal impulse to the skin acts to open pores and accelerate clearing of the blemish. In some cases, multiple treatments in one session may be necessary to effectively treat the blemish. In this case the minimum time between treatments is controlled by the circuit, which prevents misuse and possible burns. It may also be necessary to perform multiple treatments through the course of a day, or week to treat some blemishes.

In another embodiment of the handheld acne treatment device the control electronics have a mode of operation in which the thin film resistor is pulsed with multiple current pulses to produce a desired heating profile. For example, the device is first pulsed to a high temperature as previously described; one to five seconds later a second pulse of possibly lower current is produced to reheat the thin film resistor. Additional pulses can be produced with user selected delay between the pulses and variable peak temperature (or energy). Using multiple pulses can allow the user to achieve higher temperatures below the skin without burning the skin.

Not intending to limit the mechanism of action - the present invention envisions a plurality of skin improvement effects by the methods.

- By depositing a controlled amount of thermal energy at the surface and allowing the energy to flow into the upper layer of the dermis to achieve controlled damage to the collagen in the upper dermal layer. Possibly a cooling element can be activated after a predetermined time of surface heating to remove thermal energy from the surface of the skin, protect the surface of the skin from a lengthy exposure to thermal energy, and reverse the flow of thermal energy from deeper lying layers in the dermis back to the surface.
- By temporarily enlarging skin surface pores and allowing cleansing of the pores and causing expulsion of unwanted debris, dirt, and contaminants thus resulting in reduced pore size.
- By temporarily enlarging skin surface pores thus allowing nutrients, conditioners, and possibly medications to flow into deeper layers of the skin.
- By temporarily enlarging skin surface pores and allowing the expulsion of harmful sebum and bacteria thus reducing the chance for the development of acne and other sebaceous gland related ailments.
- By thermally damaging the surface layers of the skin followed by flaking and removal of portions of the stratum conium, and portions of the epidermis and dermis.

By thermally damaging vascular or a pigmented component of the skin near the skin surface (in the epidermis or upper dermis). These unwanted damaged components will then be removed by the body as waste products eliminating disfiguring skin blemishes.

The devices described herein are suitable for use in the treatment of various skin conditions and lesions.

Examples of such skin conditions and lesions are provided herein, but are not limited to the conditions and lesions described herein. Bacterial and fungal skin infections lead to common lesions such as acne, pimples and under-nail fungal infections. Other lesions are caused by irritants, which may be introduced as a result of bug bites or by exposure to other natural or man-made skin irritants. Still other skin lesions are caused by viral infection, a common example being the lesions known as "cold sores" or "fever blisters."

Other skin conditions include pustular eruptions, localized abscessed formation and local inflammatory conditions of the dermis and epidermis. One of the most common afflictions of this type are lesion caused by the condition known as acne vulgaris. Acne vulgaris is associated with the Gram-positive anaerobic bacterium, Propionibacterium acnes. Abscess formation can also occur from a number of primarily bacterial species (commonly Staphylococcus and Streptococcus) as well as fungal species, such as dermatophytes.

A further type of skin lesions are viral skin lesions such as cold sores, also known as fever blisters. Cold sores are usually caused by strains of the Herpes Simplex virus and commonly result in lesions on and near the lips and inside the mouth of an infected individual.

A further type of skin lesion are fungal infections, also known as fungal dermatitis, including conditions known medically as Tinea corporis, Tinea pedis, Tinea unguium, Tinea capitis, Tinea cruris, and Tinea barbae. Particularly troublesome is the condition known as Tinea unguium which is a fungal infection occurring under toenails or fingernails, a condition also referred to medically as onychomycosis or ringworm of the nails. Onychomycosis may be caused by several types of fungi, including Trichophyton mentagrophytes, Candida albicans or Trichophyton rubrum.

Tinea corporis, also known as tinea circinata or tinea glabrosa and referred to generally as ringworm of the body, is a fungal infection or dermatophytosis of the glabrous skin, i.e., areas of skin other than bearded area, scalp, groin, hands and feet, generally caused by fungal species such as those of Microsporum such as Microsporum canis, Trichophyton such as Trichophyton rubrum, T. Mentagrophytes, and Epidermophyton, particularly by the fungal species of Trichophyton and Epidermophyton. The condition generally includes the presence of one or more well-demarcated erythematous, scaly mascules with slightly raised borders and central healing, producing annular outlines. Various other types of lesions may also occur, such as those that are vesicular, eczematous, psoriasiform, verrucous, plaque-like, or deep.

Tinea cruris, also referred to generally as "jock itch" or ringworm of the groin, is a fungal infection or dermatophytosis of the groin, perineum and perineal regions, generally seen in males, and sometimes spreading to contiguous areas, generally caused by fungal species such as those of Microsporum, Trichophyton, and Epidermophyton, particularly by the fungal species of Trichophyton and Epidermophyton. The condition generally includes severely pruritic, sharply demarcated lesions with a raised erythematous margin and thin, dry scaling. Tinea cruris often accompanies tinea pedis (also known as "athlete's foot").

Tinea pedis results in interdigital lesions. Athlete's foot is an itching, malodorous, uncomfortable disorder resulting from large numbers of ordinary, nonvirulent bacteria proliferating in the fungus infected interspace.

Certain insect bites and contact with certain plants can expose skin to irritants that result in an itchy or painful immune response. The symptoms generally manifest soon after the introduction of the irritant, but can persist of sporadically reoccur for extended periods of time when the irritant is not effectively removed or inactivated by the immune response.

The invention relates devices for the treatment of skin conditions and skin lesions involving the application of thermal energy to the infected or irritated tissue. A skin condition or skin lesion that can be treated is any infected or irritated tissue that can be effectively treated by the application of heat.

The lesions can be the result of infection by a bacterial strain including but not limited to strains such as Propionibacterium acnes, Staphylococcus species or Streptococcus species. The present invention provides devices for the treatment of skin lesions such as the kind commonly associated with acne vulgaris. These skin lesions include pustular eruptions and localized abscesses such as cysts, nodules, pustules, papules, comedones (blackheads) and the like. These lesions include those that are commonly referred to as pimples, whiteheads, zits, acne and the like.

Alternatively or additionally, the lesions can further be result of infection by fungal species, including but not limited to fungal species capable of producing conditions such as toenail or fingernail infections, ringworm and the like. These fungal species include Microsporum species such as Microsporum canis, Trichophyton species such as Trichophyton rubrum, Trichophyton. Mentagrophytes, Epidermophyton species, Candida albicans, and the like. Such fungal species are sometimes referred to broadly as "dermatophytes".

Alternatively or additionally, in other embodiments, the skin lesions can be the result of viral infections, including infections caused by Herpes viruses such as Herpes simplex types I and II (cold sores and genital herpes), Varicella zoster (chicken pox) and the like.

Alternatively or additionally, embodiments of the present invention provide devices for the application of heat for the treatment of skin lesions caused by an irritant. Common skin irritants that can be treated by the present invention include those introduced by bug bites, such as mosquito, chigger, ant, spider bites, scabies and the like. Other skin irritants introduced by other animal species, such as jellyfish, snakes and the like, or by plants such as poison ivy, poison oak, poison sumac and the like, can also be treated using the devices of the present invention.

EXAMPLE

TREATMENT OF ACNE WITH HEAT:

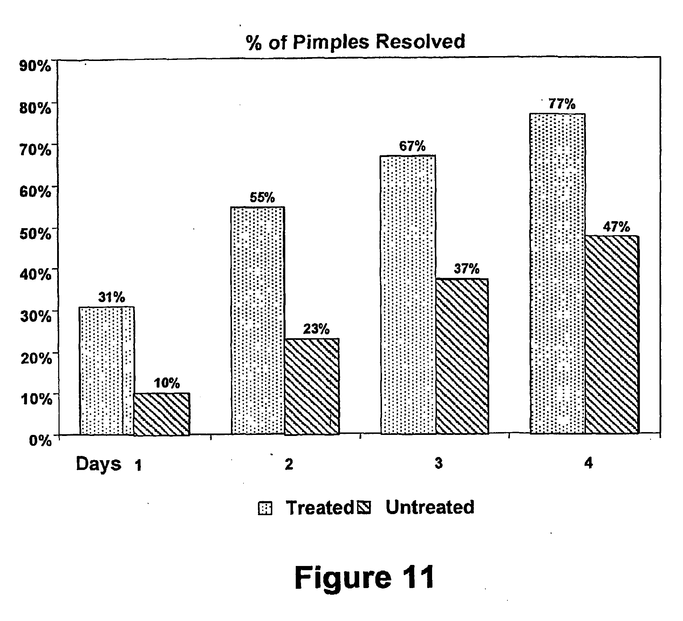

Figure 11 shows the results of a clinical trial in which the device was used to treat mild to moderate acne. The study consisted of tracking the progress of treated and untreated lesions (pimples) over a 5 day period. For each patient the treated and untreated lesions were randomly selected. To treat a lesion the device was applied once a day, each day to the pimple and a single pulse of heat applied. It is believed that the peak temperature applied by the device was about 250°C, with this temperature decaying to approximately 100°C in less than 0.5 seconds. The temperature ramps up in approximately 1 millisecond. After completion of treatment on day 5, two separate investigators independently assessed the subject's overall clinical progress and determined any adverse events. Clinical evaluation was determined by documentation of the patient's acne lesions, comparing the subject's before treatment facial photos to each subsequent treatment day as well as the final 5th day facial photos. Total number of lesions, severity, and location was documented, graded, and compared. Grading was done on a point system Each individual facial lesion was assigned a number to grade each lesion. The following outlines the grading point system used for each individual acne lesion:

- 1 (one) point - Mild lesion - level with surface or skin or minimally raised lesion, minimal erythema.
- 2 (two) points - Moderate lesion - visible lesion, raised 1-2mm from skin plane, moderate erythema (0.5 mm - 1 mm), with or without fluctuance.
- 3 (three) points - Severe lesion - visible lesion, raised 2 mm or more, erythema extension >1mm, with or without fluctuance, open or closed, may or may not be draining serous fluid.

The final score was the sum total of all graded individual lesions. The score was determined from the baseline on the first day, before treatment. Each day of treatment, all individual acne lesions were graded and the sum total determined as the patient's score for that day. The final score was determined after treatment on the final (5th) day from the sum total grade of all individual lesions. A score of 0-1 for a lesion indicated the lesion was resolved.
The results shown in Figure 11 show that treated pimples resolve faster then untreated pimples.

The present invention can also be combined with topical gels or creams to improve treatment of acne. For example, topical gel with benzoyl peroxide can be applied after treatment with the device. For optimum results the area to be treated is first washed with mild soap or cleanser. After washing the area to be treated, the device is applied a minimum of one time and then a topical acne gel is applied. This process would usually be repeated twice a day.

The above descriptions and illustrations are only by way of example and are not to be taken as limiting the invention in any manner. One skilled in the art can substitute known equivalents for the structures and means described. The full scope and definition of the invention, therefore, is set forth in the following claims.

**Claims**

1.  A device for treatment of skin conditions comprising:

    a heating element adapted and configured to contact a skin surface wherein
    the heating element includes a resistive heater;
    a capacitor that can be discharged through the heating element in response to a user input,
    and a controller adapted and configured to automatically heat the heating element to a temperature of at least about 70°C in a period of less than about one second and to maintain the heating element at a temperature of at least about 70°C for a period of less than one second in response to the user input, wherein the values of the resistive heater and capacitor are selected so that the energy released by the resistive heater can be kept below the threshold for tissue burn.

2.  The device of claim 1 wherein the controller is further adapted to cease heating the heating element and to reheat the heating element without a further user input.

3.  The device of claim 1 or 2, wherein said heating element and controller are located in a housing, said housing being capable of being held in a hand of a user.

4.  The device of claim 1, 2 or 3, wherein a user interface is adapted and configured to be activated by a user to provide said user input.

5.  The device of one of the preceding claims, wherein the heating circuit is adapted and configured to heat the heating element to a temperature of at least about 67°C for less than about 0.1 second.

6.  The device of claim 5, wherein said resistive heater is a thin metal resistor.

7.  The device of one of the preceding claims, wherein said heating element comprises a metal selected from the group consisting of nichrome, tungsten, aluminum, gold, copper and/or steel.

8.  The device of one of the preceding claims, wherein said heating element comprises a backing layer, an electrically insulating protective layer, and/or a high conductivity layer.

9.  The device of one of the preceding claims, wherein a skin contact surface of said heating element has a surface area of about 1 cm$^2$.

10. The device of one of the preceding claims, wherein said temperature is about 75°C - about 100°C.

11. The device of one of the claims 1 to 10, wherein said temperature is about 100°C about 200°C.

12. The device of one of the preceding claims, wherein said temperature is achieved in a period of less than about 0.5 seconds.

13. The device of one of the preceding claims, wherein the total energy transferred by the device is limited to less than about 10 J/cm$^2$.

14. The device of one of the preceding claims, wherein the total energy transferred by the device is limited to less than about 5 J/cm$^2$.

15. The device of one of the preceding claims, wherein said temperature limits total energy transferred to the skin to less than about 50 J/cm$^2$.

16. The device of one of the preceding claims, wherein said temperature limits total energy transferred to the skin to less than about 5 J/cm$^2$.

17. The device of one of the preceding claims, further comprising a temperature sensor, said sensor being adapted and configured to sense the temperature of said heating element and to control said temperature of said heating element.

18. The device of one of the preceding claims, further comprising a cooling element, said cooling element adapted and configured to cool said heating element and/or a skin surface to a temperature of less than about 60°C.

19. The device of one of the preceding claims, further comprising a light emitting diode.

20. The device of claim 5, wherein the heating circuit is further adapted to heat the heating element to a temperature of at least 67°C for less than about 0.1 second, lower the temperature of the heating element for a period of about 0.5 seconds, and reheat the heating element to a temperature of at least 67°C for less than about 0.1 second.

21. The device of one of the preceding claims, wherein activation of said user interface by a user heats said heating element to a peak temperature of about 250°C in about 1 millisecond and said peak temperature decays to about 100°C in less than about 0.5 seconds.

22. The device of one of the preceding claims wherein the maximum energy which is discharged from the capacitor through the thin resistive heater amounts to 0,5*C*V*V, wherein C is the capacitor capacitance and V is the final voltage across the capacitor.

**Patentansprüche**

1. Vorrichtung zur Behandlung von Hauterkrankungen, mit:

   einem Heizelement, welches so eingerichtet und konfiguriert ist, dass es mit einer Hautoberfläche in Kontakt kommt, wobei das Heizelement einen Heizwiderstand enthält;
   einem Kondensator, der in Reaktion auf eine Benutzereingabe durch das Heizelement entladen werden kann;
   sowie einer Steuereinrichtung, die so eingerichtet und konfiguriert ist, dass sie das Heizelement in einem Zeitraum von weniger als etwa einer Sekunde automatisch auf eine Temperatur von mindestens etwa 70°C erhitzt und in Reaktion auf die Benutzereingabe das Heizelement während eines Zeitraumes von weniger als einer Sekunde auf einer Temperatur von mindestens etwa 70°C hält, wobei die Werte des Heizwiderstands und des Kondensators so ausgewählt werden, dass die von dem Heizwiderstand freigesetzte Energie unterhalb des Grenzwertes für eine Gewebeverbrennung gehalten werden kann.

2. Vorrichtung nach Anspruch 1, wobei die Steuereinrichtung weiterhin so eingerichtet ist, dass sie das Erhitzen des Heizelements beendet und das Heizelement ohne eine weitere Benutzereingabe wieder erhitzt.

3. Vorrichtung nach Anspruch 1 oder 2, wobei das Heizelement und die Steuereinrichtung in einem Gehäuse angeordnet sind, wobei das Gehäuse in der Hand eines Benutzers gehalten werden kann.

4. Vorrichtung nach Anspruch 1, 2 oder 3, wobei eine Benutzerschnittstelle so eingerichtet und konfiguriert ist, dass sie von einem Benutzer aktiviert wird, um die Benutzereingabe vorzusehen.

5. Vorrichtung nach einem der vorangegangenen Ansprüche, wobei der Heizstromkreis so eingerichtet und konfiguriert ist, dass das Heizelement für weniger als etwa 0,1 Sekunden auf eine Temperatur von mindestens etwa 67°C erhitzt wird.

6. Vorrichtung nach Anspruch 5, wobei der Heizwiderstand ein dünner Metallwiderstand ist.

7. Vorrichtung nach einem der vorangegangenen Ansprüche, wobei das Heizelement ein Metall umfasst, welches aus der Gruppe, bestehend aus Nickel-Chrom, Wolfram, Aluminium, Gold, Kupfer und/oder Stahl, ausgewählt wird.

8. Vorrichtung nach einem der vorangegangenen Ansprüche, wobei das Heizelement eine Rückschicht, eine elektrisch isolierende Schutzschicht und/oder eine Schicht mit hoher Leitfähigkeit umfasst.

9. Vorrichtung nach einem der vorangegangenen Ansprüche, wobei eine Hautkontaktoberfläche des Heizelements einen Oberflächenbereich von etwa 1 cm$^2$ aufweist.

10. Vorrichtung nach einem der vorangegangenen Ansprüche, wobei die Temperatur etwa 75°C bis etwa 100°C beträgt.

11. Vorrichtung nach einem der Ansprüche 1 bis 10, wobei die Temperatur etwa 100°C bis etwa 200°C beträgt.

12. Vorrichtung nach einem der vorangegangenen Ansprüche, wobei die Temperatur in einem Zeitraum von weniger als etwa 0,5 Sekunden erreicht wird.

13. Vorrichtung nach einem der vorangegangenen Ansprüche, wobei die durch die Vorrichtung übertragene Gesamtenergie auf weniger als etwa 10 J/cm$^2$ begrenzt wird.

14. Vorrichtung nach einem der vorangegangenen Ansprüche, wobei die durch die Vorrichtung übertragene Gesamtenergie auf weniger als etwa 5 J/cm$^2$ begrenzt wird.

15. Vorrichtung nach einem der vorangegangenen Ansprüche, wobei die Temperatur die auf die Haut übertragene Gesamtenergie auf weniger als etwa 50 J/cm$^2$ begrenzt.

16. Vorrichtung nach einem der vorangegangenen Ansprüche, wobei die Temperatur die auf die Haut übertragene Gesamtenergie auf weniger als etwa 5 J/cm$^2$ begrenzt.

17. Vorrichtung nach einem der vorangegangenen Ansprüche, die weiterhin einen Temperatursensor umfasst, wobei der Sensor so eingerichtet und konfiguriert ist, dass er die Temperatur des Heizelements abtastet und die Temperatur des Heizelements steuert.

18. Vorrichtung nach einem der vorangegangenen Ansprüche, welche weiterhin ein Kühlelement umfasst, wobei das Kühlelement so eingerichtet und konfiguriert ist, dass es das Heizelement und/oder eine Hautoberfläche auf eine Temperatur von weniger als etwa 60°C abkühlt.

19. Vorrichtung nach einem der vorangegangenen Ansprüche, welche weiterhin eine Licht emittierende Diode umfasst.

20. Vorrichtung nach Anspruch 5, wobei der Heizstromkreis weiterhin so eingerichtet ist, dass er das Heizelement für weniger als etwa 0,1 Sekunden auf eine Temperatur von mindestens 67°C erhitzt, die Temperatur des Heizelements für einen Zeitraum von etwa 0,5 Sekunden verringert und das Heizelement für weniger als etwa 0,1 Sekunden wieder auf eine Temperatur von mindestens 67°C erhitzt.

21. Vorrichtung nach einem der vorangegangenen Ansprüche, wobei durch die Aktivierung der Benutzerschnittstelle durch einen Benutzer das Heizelement in etwa 1 Millisekunde auf eine höchste Temperatur von etwa 250°C erhitzt wird und diese höchste Temperatur in weniger als etwa 0,5 Sekunden auf etwa 100°C abfällt.

22. Vorrichtung nach einem der vorangegangenen Ansprüche, wobei die maximale Energie, die von dem Kondensator durch den dünnen Heizwiderstand entladen wird, 0,5*C*V*V beträgt, wobei C die Kondensatorkapazität und V die Endspannung an dem Kondensator darstellt.

## Revendications

1. Dispositif de traitement de conditions de la peau comprenant :

   un élément chauffant qui est adapté et configuré de manière à entrer en contact avec une surface de la peau, dans lequel l'élément chauffant comprend un dispositif de chauffage à résistance électrique ;
   un condensateur qui peut être déchargé par le biais de l'élément chauffant en réponse à une entrée d'utilisateur ; et
   et un contrôleur qui est adapté et configuré de manière à chauffer automatiquement l'élément chauffant jusqu'à une température d'au moins environ 70°C pendant une période de moins d'environ une seconde et de manière à maintenir l'élément chauffant à une température d'au moins environ 70°C pendant une période d'au moins une seconde en réponse à l'entrée d'utilisateur, dans lequel les valeurs du dispositif de chauffage à résistance électrique et du condensateur sont sélectionnées de telle façon que l'énergie qui est dégagée par le dispositif de chauffage à résistance électrique puisse être maintenue au-dessous du seuil en vue de la brûlure de tissus.

2. Dispositif selon la revendication 1, dans lequel le contrôleur est en outre adapté de manière cesser le chauffage de l'élément chauffant et de manière à réchauffer l'élément chauffant sans une nouvelle autre entrée d'utilisateur.

3. Dispositif selon la revendication 1 ou selon la revendication 2, dans lequel ledit élément chauffant et ledit contrôleur se situent dans un boîtier, ledit boîtier étant capable d'être tenu dans une main d'un utilisateur.

4. Dispositif selon les revendications précédentes 1, 2 ou 3, dans lequel une interface utilisateur est adaptée et configurée de manière à être activée par un utilisateur afin de fournir ladite entrée d'utilisateur.

5. Dispositif selon une des revendications précédentes 1 à 4, dans lequel le circuit de chauffage est adapté et configuré de manière à chauffer l'élément chauffant jusqu'à une température d'au moins environ 67°C pendant moins d'environ 0,1 seconde.

6. Dispositif selon la revendication 5, dans lequel ledit dispositif de chauffage à résistance électrique est une résistance métallique mince.

7. Dispositif selon une des revendications précédentes 1 à 6, dans lequel ledit élément chauffant comprend un métal qui est sélectionné parmi le groupe constitué de nichrome, de tungstène, d'aluminium, d'or, de cuivre et/ou d'acier.

8. Dispositif selon une des revendications précédentes 1 à 7, dans lequel ledit élément chauffant comprend une couche de support, une couche protectrice électriquement isolante et/ou une couche de haute conductivité.

9. Dispositif selon une des revendications précédentes 1 à 8, dans lequel une surface de contact de la peau dudit élément chauffant présente une zone de surface de l'ordre de 1 cm$^2$.

10. Dispositif selon une des revendications précédentes 1 à 9, dans lequel ladite température se situe dans la gamme comprise entre environ 75°C et environ 100°C.

11. Dispositif selon une des revendications précédentes 1 à 10, dans lequel ladite température se situe dans la gamme comprise entre environ 100°C et environ 200°C.

12. Dispositif selon une des revendications précédentes 1 à 11, dans lequel ladite température est obtenue pendant une période de moins d'environ 0,5 secondes.

13. Dispositif selon une des revendications précédentes 1 à 12, dans lequel l'énergie totale qui est transférée par le dispositif est limitée à moins d'environ 10 J/cm$^2$.

14. Dispositif selon une des revendications précédentes 1 à 13, dans lequel l'énergie totale qui est transférée par le dispositif est limitée à moins d'environ 5 J/cm$^2$.

15. Dispositif selon une des revendications précédentes 1 à 14, dans lequel ladite température limite l'énergie totale qui est transférée à la peau à moins d'environ 50 J/cm$^2$.

16. Dispositif selon une des revendications précédentes 1 à 15, dans lequel ladite température limite l'énergie totale qui est transférée à la peau à moins d'environ 5 J/cm$^2$.

17. Dispositif selon une des revendications précédentes 1 à 16, comprenant en outre un capteur de température, ledit capteur étant adapté et configuré de manière à capter la température dudit élément chauffant et de manière à commander ladite température dudit élément chauffant.

18. Dispositif selon une des revendications précédentes 1 à 17, comprenant en outre un élément de refroidissement, ledit élément de refroidissement étant adapté et configuré de manière à refroidir ledit élément chauffant et/ou une surface de la peau jusqu'à une température de moins d'environ 60°C.

19. Dispositif selon une des revendications précédentes 1 à 18, comprenant en outre une diode électroluminescente.

20. Dispositif selon la revendication 5, dans lequel le circuit de chauffage est en outre adapté de manière à chauffer l'élément chauffant jusqu'à une température d'au moins 67°C pendant moins d'environ 0,1 seconde, de manière à abaisser la température de l'élément chauffant pendant une période de l'ordre de 0,5 secondes et de manière à réchauffer l'élément chauffant jusqu'à une température d'au moins 67°C pendant moins d'environ 0,1 seconde.

21. Dispositif selon une des revendications précédentes 1 à 20, dans lequel l'activation de ladite interface utilisateur par un utilisateur chauffe ledit élément chauffant jusqu'à une température de crête de l'ordre de 250°C en environ 1 milliseconde et dans lequel ladite température de crête décroît jusqu'à environ 100°C en moins d'environ 0,5 secondes.

22. Dispositif selon une des revendications précédentes 1 à 21, dans lequel l'énergie maximale qui est déchargée à partir du condensateur par le biais du dispositif de chauffage mince à résistance électrique se monte à 0,5*C*V*V où C est la capacité du condensateur et où V est la tension finale à travers le condensateur.

**Figure 1**

**Figure 2**

**Figure 3**

**Figure 4**

**Figure 5**

Figure 6

**Figure 7**

EP 1 830 730 B1

Figure 8

Figure 9

10

**Figure 10**

**Figure 11**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6635075 B, Li


**Non-patent literature cited in the description**

- **F.C. Henriques.** Studies of Thermal Injury: The Predictability and the Significance of Thermally Induced Rate Processes Leading to Irreversible Epidermal Injury. *Archives of Pathology,* 05 May 1947, vol. 43, 489-502